# EUROPEAN PATENT SPECIFICATION

(11) **EP 4 583 962 B1**
(45) Date of publication and mention of the grant of the patent: **08.07.2026**
(21) Application number: 23739619.7
(22) Date of filing: 19.07.2023
(51) Int. Cl.: A61N 1/02, A61N 1/05, A61N 1/36

(54) **MULTI-ELECTRODE NEUROSTIMULATION SYSTEM FOR ALTERATION OF NEURAL FUNCTION**
MULTIELEKTRODEN-NEUROSTIMULATIONSSYSTEM ZUR VERÄNDERUNG NEURONALER FUNKTIONEN
SYSTÈME DE NEUROSTIMULATION À ÉLECTRODES MULTIPLES POUR L'ALTÉRATION DE LA FONCTION NEURONALE

(30) Priority: 07.09.2022 US 202263404427 P; 27.10.2022 EP 22204025
(43) Date of publication of application: 16.07.2025
(73) Proprietor: BIOTRONIK SE & Co. KG, 12359 Berlin (DE)
(72) Inventor: BARU, Marcelo, Tualatin, Oregon 97062 (US); KIBLER, Andrew B., Lake Oswego, Oregon 97035 (US)
(74) Representative: Biotronik Corporate Services SE
(86) International application number: PCT/EP2023/070014
(87) International publication number: WO 2024/051993

(56) References cited:
- EP-A1- 3 381 507
- US-A1- 2014 243 924
- US-A1- 2017 050 024
- US-A1- 2018 280 691
- US-A1- 2019 105 502
- US-A1- 2019 329 039
- US-A1- 2020 376 263

## Description

The present invention relates to a neurostimulation system.

Delivering stimulation via multiple electrodes across location and time to improve pain relief is known in the prior art. However, regarding these stimulation techniques, it remains a challenge to electrically mimic the effects that local anaesthetics have in blocking the propagation of pain-related signals. It is further known that the application of direct current of sufficient amplitude (DC) blocks the conduction of nerve action potentials and may be effective at reducing comfortable and painful sensory sensations. However, such electrical therapy would not be suitable chronically given the electrode corrosion and nerve damage associated with it.

Recently, ultra-low frequency (sub Hz) biphasic current waveforms, injected via implanted high-charge capacity electrodes, have been disclosed and claim to overcome the limitations of DC application. Some of these therapies denominated charge-balanced direct current (CBDC) or Carousel utilize multiple electrodes and different duty cycles to alleviate the amount of charge required per electrode to maintain conduction block, cycling the CBDC through each electrode is also possible. Ultra-low-frequency therapies in prior art utilize a charge balance scheme that is implemented independently for each electrode which complicates implementation and particularly utilizes an auxiliary electrode for therapy delivery. However, no disclosure exists for implementation of such CBDC or Carousel therapy in a single-fault safe system such as an implantable pulse generator (IPG).

Furthermore, conventional tonic-based stimulation, High Frequency, burst pulse therapy, pulsed stimulation pattern, differential target multiplexing (DTM), FAST (and related),

Multiphase therapy, and charge balanced DC (CBDC) or Carousel waveform stimulation (block) are known in prior art, see for instance EP 338 150 7 A1.

Furthermore, particularly, US10,195,434B2 describes systems and methods for the treatment of pain using electrical nerve conduction block (ENCB).

US 2020/376263 A1 teaches an electrical stimulation system includes at least one electrical stimulation lead, each of the at least one electrical stimulation lead including a plurality of stimulation electrodes; and a processor coupled to the lead and configured to perform actions, including: directing delivery of at least one electrical pulse through at least one of the stimulation electrodes of the at least one electrical stimulation lead to tissue of a patient; and directing discrete or intermittent measurement of an electrical characteristic of the tissue using at least one of the stimulation electrodes of the at least one electrical stimulation lead during, and after, delivery of the at least one electrical pulse to the tissue of the patient.

US 2019/0105502 A1 describes an implantable medical device for implantation into a patient that includes a housing, a pulse generation circuit disposed at least partially within the housing, a plurality of electrodes electrically coupled to the pulse generation circuit, the plurality of electrodes being exposed external to the housing, and a controller operatively coupled to the pulse generation circuit. The controller may be configured to command the pulse generation circuit to deliver a phasic conducted communication pulse via at least two of the plurality of electrodes.

US 2018/0280691 A1 describes systems and methods for nerve conduction block. The systems and methods can utilize at least one renewable electrode. The methods can include delivering a first direct current with a first polarity to an electrode proximate nervous tissue sufficient to block conduction in the nervous tissue. Delivering the first direct current can place the nervous tissue in a hypersuppressed state at least partially preventing conduction of the nervous tissue after cessation of delivering of the first direct current. The nervous tissue can be maintained in the hypersuppressed state for a desired period, such as at least about 1 minute.

Furthermore, US9,498,621B2 teaches ionic coupling electrodes, wherein a first portion can be coupled to a single-phase current source and carries current flow via electrons. The electrode can include a second portion to apply a current to a nerve tissue, wherein the second portion carries current flow via ions.

The current technique of delivering quasi-DC stimulation to the brain includes transcranial direct current stimulation, using sponge-like wet electrodes placed on the head of a participant. These are temporary electrodes which require manual preparation and placement every time they are used. The resulting electric field is highly diffuse and nonspecific to its neuromodulation target due to interference from the relatively resistive nature of skull, and limitations on surface electrode placement.

Further, existing peripheral neuromodulation solutions include use of high area electrodes, for example, conductively coated electro-spun materials, and common long pulse width bipolar stimulation pulses.

Spinal cord stimulation (SCS), as a means of pain relief for patients suffering from neuropathic pain, has traditionally been practiced as a therapy requiring paresthesia sensations to overlap a patient's region of pain in order to provide relief. In an attempt to improve outcomes associated with traditional paresthesia-based SCS, several alternative "paresthesia-free" stimulation waveform paradigms where later introduced in the market such as High Density, burst pulse therapy, High Frequency, and DTM.

Although paresthesia-free therapies known in prior art claim improved efficacy relative to conventional tonic-based SCS, it remains a challenge to electrically mimic the effects that local anaesthetics have in blocking the propagation of pain-related signals. Furthermore, SCS therapies often rely on electrical pulses with pulse widths in the range of tens of µs to 1 ms which are typically delivered at frequencies ranging from tens of Hz to 10 kHz. While nerve conduction blocking is possible at high frequencies of stimulation as a mechanism of action for the treatment of pain, for power consumption reasons this block is preferably delivered at much lower frequencies, preferably sub Hz, with very long (seconds) equivalent pulse widths.

SCS may also be used as a heart failure treatment, through neuromodulation of the autonomic nervous system, and paralysis treatment, by exciting dormant sensory and musculoskeletal reflex pathways, and the same technologies for delivering stimulation are used.

Nerve conduction blocking using direct current (DC) has been shown in prior art but it is not common as a chronic therapy since the challenge of high risk of electrode corrosion and resulting nerve damage must be overcome with specialized electrodes. Monophasic stimulation, where a constant current is passed for a period of time and then the stimulator circuit is open circuited until the next pulse, has also been proposed in the prior art but it can suffer from similar issues as DC as electrode potential is not brought back towards the equilibrium potential by a charge balance phase which can cause accumulation of unrecoverable charge during the open-circuit inter-pulse interval.

Further, charge-balanced DC (CBDC) or Carousel waveform stimulation has recently been proposed to overcome the limitations of DC or monophasic nerve conduction blocking (Vrabec et al "Continuous Direct Current Nerve Block Using Multi Contact High Capacitance Electrodes", IEEE Transactions on Neural Systems and Rehabilitation Engineering, vol. 25, no. 6, June 2017). Here, a monopolar CBDC block configuration may be employed with multiple electrodes and a common reference placed remotely in tissue. In this configuration, the region of blocking changes as the current shifts from one electrode to the next. Also, the Carousel waveform requires at least two stimulus currents to be delivered concurrently at different sites within a patient's body which presents implementation challenges for an implantable pulse generator (IPG) as both current generators are referenced to the battery of the IPG. A possible solution would be to have two or more entirely separate generators, powered by separate batteries. This is however impractical and difficult to implement in the context of clinical IPGs that seek to reduce size.

Another solution that has been traditionally used in external electrical stimulators, and proposed for example in US 8,977,363, is the use of transformer isolation to electrically float the current generators. This may also be difficult to realize in an IPG, primarily due to the size of the transformers.

Further, US2019/0314630A1 discloses systems and methods for nerve conduction block, which can include delivering a first direct current with a first polarity to an electrode proximate nervous tissue sufficient to block conduction in the nervous tissue. Particularly, delivering ultra-low-frequency therapy is described in US2019/0314630A1 based on an electrode-ion current conversion cell (EICCC), e.g., Ag/AgCl. Particularly, TiN can be used as high-charge capacity electrode. However, TiN electrodes may suffer from long-term stability as charge starts to be transferred via Faradic reactions rather than in a capacitive manner. This is likely due to anodic oxidation of the TiN surface.

Based on the above, it is an objective of the present invention to provide a practical low-frequency electrical neuromodulation system which is able to alter transmembrane potentials of target cells for extended periods of time, particularly in the range from 1 ms to 10 s.

This problem is solved by a neurostimulation device having the features of claim 1. Preferred embodiments of these aspects of the present invention are stated in the corresponding independent claims and/or are described below.

According to claim 1 a neurostimulation device for providing neurostimulation is disclosed, the device comprising a plurality of Z electrodes, wherein Z is an integer number and equal or larger than 3, and a programmable pulse generator configured to deliver in a cycle via each electrode of a group of N electrodes of said plurality of Z electrodes, wherein N is an integer number being smaller or equal to Z, and wherein N is equal to or larger than 3, wherein N is equal to 3 in case Z is equal to 3, a set of electric pulses including one therapeutic electric pulse, and a number of N-1 charge balancing pulses, wherein the charge balancing electric pulses each have a polarity that is opposite a polarity of the therapeutic electric pulse, and
wherein the integrated average current delivered by the therapeutic electric pulse and charge balancing pulses is zero over time; at least one of the electric pulses comprises a pulse shape comprising
- a rising phase having a duration of at least 0.1s,
- a plateau phase having a duration of at least 0.1 s, and
- a falling phase having a duration of at least 0.1s.

According to an embodiment, said pulse shape starts with the rising phase, followed by a plateau phase, followed by a falling phase for an anodic pulse. Alternatively, said pulse shape starts with the falling phase, followed by a plateau phase, followed by a rising phase for a cathodic pulse.

According to an embodiment, each electrode undergoes a therapeutic electric pulse in a cyclic fashion (period T) with charge balancing pulses of opposite polarity. Moreover, the current of each therapeutic electric pulse is substantially returned by the charge balancing pulses in the other N-1 electrodes.

Particularly, the neurostimulation system according to the present invention allows to deliver multiphase SCS therapy that employs nerve conduction block as a possible mechanism of action for pain mitigation. Employing a suitable high-capacity electrode and either a charge metering or switched DC blocking capacitor configuration for single fault protection is crucial for implementation in a chronic implant that needs to target small IPG sizes, and this can be achieved by the present invention.

According to the invention, multiphase SCS therapy is understood as stimulation comprising a plurality of Z electrodes, wherein Z is an integer number and equal or larger than 3, and a programmable pulse generator configured to deliver in a cycle via each electrode of a group of N electrodes of said plurality of Z electrodes, wherein N is an integer number being smaller or equal to Z, wherein N is equal to 3 in case Z is equal to 3, a set of electric pulses including one therapeutic electric pulse, and a number of N-1 charge balancing pulses, wherein the charge balancing electric pulses each have a polarity that is opposite a polarity of the therapeutic electric pulse.

Particularly, using the device according to the present invention an ultra-low-frequency Multiphase therapy can be provided, particularly via a traditional Pt/Ir electrode with surface restructuring, that enhances the block under adjacent electrodes, wherein the delivered stimulation is single-fault safe, and given its charge self-balancing characteristic, is suitable for chronic delivery and implementation in an implantable pulse generator without the need for an auxiliary electrode.

Particularly, the primary applications of the invention to neuromodulation include therapies where alteration of neural function are desired, including modulation of synaptic coupling, extracellular ionic concentrations, and modulation of depolarization state including blockage/inactivation of depolarization. Deep brain stimulation (DBS), spinal cord stimulation (SCS) for autonomic modulation and pain modulation, and peripheral nerve modulation all are relevant to this technology.

According to a preferred embodiment of the neurostimulation device, the rising phase has a duration being smaller or equal to 4 s, preferably smaller or equal than 2 s, wherein the plateau phase has a duration being smaller or equal to 4 s, and wherein the falling phase has a duration being smaller or equal to 4 s, preferably smaller or equal to 2 s.

Furthermore, according to an embodiment of the neurostimulation device, during the rising phase, a current level of the pulse shape (electric pulse) steadily rises from a first current amplitude to a plateau current amplitude, wherein the plateau current amplitude is in the range of 0.1 mA to 5.0 mA, and wherein during the plateau phase, the current level of the pulse shape maintains the plateau current amplitude, and wherein during the falling phase, the current level of pulse shape decreases from the plateau current amplitude to the first current amplitude.

Furthermore, according to an embodiment of the neurostimulation device, the programmable pulse generator is configured to generate at least two successive electric pulses having said pulse shape, wherein the at least two successive electric pulses are separated by an inter-pulse-interval of at least 0.1 s.

Furthermore, according to an embodiment of the neurostimulation device, the inter-pulse-interval is smaller or equal than 4 s, preferably smaller or equal to 2 s.

Further, according to yet another embodiment of the neurostimulation device, the programmable pulse generator is configured to generate an inter-pulse-interval of between each electric pulse of said set of electric pulses, wherein the respective inter-pulse-interval is in the range from 0.1 s to 4 s, preferably 0.1 s to 2 s.

Further, in an embodiment of the neurostimulation device, the neurostimulation device is configured to measure a total charge injected during a therapeutic electric pulse (TEP) and to compare it to a limit value, particularly for preventing electrode corrosion.

Furthermore, according to an embodiment of the neurostimulation device, for measuring said total charge, the neurostimulation device is configured to sense an electric current of the therapeutic pulse using a resistor having a resistance R and a further resistor having a resistance n*R, wherein n is an integer number, and a voltage converted by a voltage-to-current converter to a further current being equal to said electric current divided by said integer number n, wherein the neurostimulation device is further configured to integrate said further current to generate an output corresponding to said total charge, and to compare the output to said limit value.

Furthermore, according to an embodiment of the present invention, the neurostimulator is configured to perform a charge per charge delivery phase of over 12 uC to an electrode by a charge delivery circuit. Said charge delivery circuit comprises at least two charge blocking capacitors which are sequentially charged and discharged so as to deliver a desired net charge output to said stimulating electrode during the charge delivery phase. For instance, the duration of time of each capacitor charging and discharging ranges between 10 microseconds and 100 microseconds, or between 1 microsecond and 500 microseconds.

Furthermore, according to an embodiment of the neurostimulation system, the neurostimulation device is configured to disconnect current sources providing the charge balancing electric pulses and to declare a fault to prevent further stimulation.

Furthermore, according to an embodiment of the neurostimulation device, for providing a therapeutic electric pulse by means of an electrode of said group of N electrodes, said electrode is tied to system ground (e.g. negative terminal of the IPG battery) and the remaining electrodes of said group of N electrodes are each connected to an associated building block, each building block comprising a first and a second blocking capacitor and circuitry that is configured to alternate in time a connection in series with the corresponding remaining electrode.

Furthermore, in an embodiment of the neurostimulation device, the neurostimulation device is configured to control the alternating connection of the blocking capacitors C₁, C₂ of each building block by a first and a second signal assuming a high value and a low value, respectively, wherein when the first signal assumes its high value, it turns on a sourcing current and a copy of it flows through second blocking capacitor which is coupled (e.g. by a switch) to the corresponding remaining electrode, while discharging the first blocking capacitor, and wherein when the second signal assumes its high value, it turns on a further sourcing current and a copy of the further sourcing current flows through first blocking capacitor which is coupled (e.g. via a switch) to the corresponding remaining electrode, while discharging the second blocking capacitor, wherein a sum of currents circulating through the remaining electrodes are returned at said electrode providing the therapeutic pulse.

One skilled in the art will recognize that additional capacitors and sourcing currents may be added, resulting in the same effect of a design which is single fault tolerant, and capable of sourcing large amounts of charge to target electrodes.
- Figure 1: shows an embodiment of a system according to the present invention;
- Figure 2: shows an embodiment of the present invention allowing single-fault charge metering;
- Figure 3: shows an embodiment of a system according to the present invention being particularly suitable for super Hz frequencies;
- Figure 4: shows an exemplary implementation of a building block shown in Fig. 3; and
- Figure 5: shows an embodiment of the present invention allowing delivery of duty cycled Multiphase therapy.

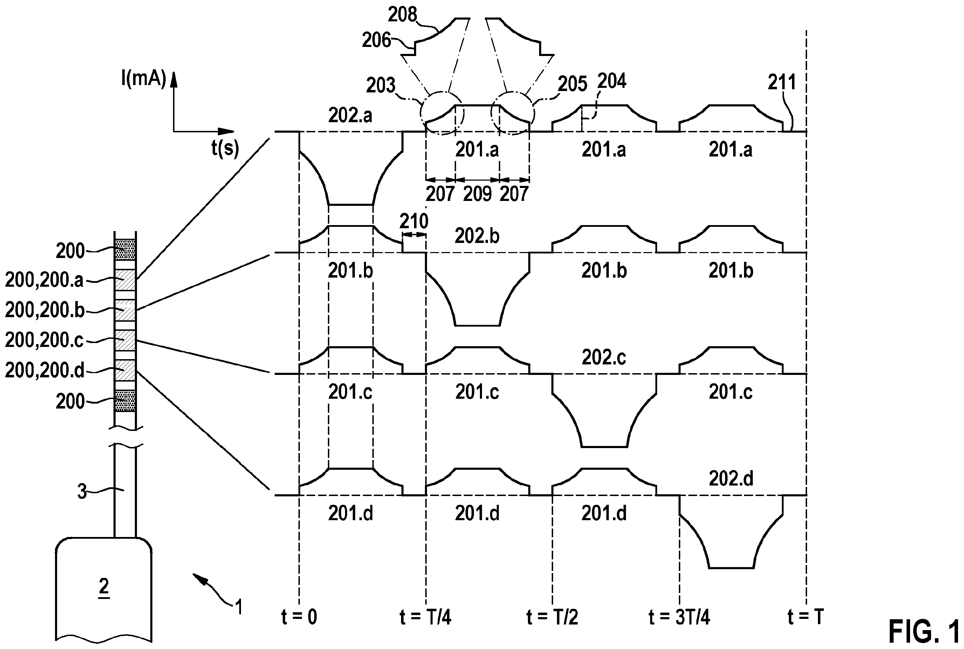

Fig. 1 illustrates an embodiment of a neurostimulation system 1 according to the present invention, comprising a plurality of Z electrodes 200, wherein Z is an integer number and equal or larger than 3, and a programmable implantable pulse generator (IPG) 2 configured to deliver, in a cycle T via each electrode 200.a, 200.b, 200.c, 200.d of a group of N electrodes of said plurality of Z electrodes 200, wherein N is an integer number being smaller or equal to Z, wherein N is equal to 3 in case Z is equal to 3, a set of electric pulses including one therapeutic electric pulse (TEP) 202.a, 202.b, 202.c., 202.d, and a number of N-1 charge balancing pulses 201.a, 201.b., 201.c. 201.d, wherein the charge balancing electric pulses each have a polarity that is opposite a polarity of the TEP 202.a, 202.b, 202.c., 202.d, and wherein at least one of the electric pulses comprises a pulse shape comprising a rising phase 203 having a duration of at least 0.1 s, followed by a plateau phase 204 having a duration of at least 0.1 s, and followed by a falling phase 205 having a duration of at least 0.1s. In the example shown in Fig. 1, N is equal to 4.

Particularly, the IPG 2 can be connected to one or more percutaneous or paddle leads 3 for spinal cord stimulation (SCS). Particularly, said electrodes 200 of the lead(s) 3 can be Pt/Ir electrodes that preferably underwent a surface restructuring process that permits injecting charges in the 15 mC range without electrode corrosion or nerve damage. Given a typical area of 12.5 mm² for a percutaneous SCS electrode, this translates into a 120 mC/cm² charge injection level. Particularly, using known hierarchical surface restructuring via ultra-short pulse lasers based on Pt10Ir electrodes, 120 mC/cm² is easily achievable.

In a preferred embodiment for SCS, as indicated in Fig. 1, a Multiphase therapy according to the present invention is preferably delivered via four adjacent electrodes 200.a, 200.b, 200.c, 200.d as described above, which are preferably spaced 1.5 mm apart according to an embodiment. Particularly, when electrode 200.a is to provide electrical nerve conduction block (ECNB), concurrent current-controlled phases 201.b, 201.c, 201.d are imposed in the other electrodes 200.b, 200.c, 200.d and those returned in electrode 200.a generating the ENCB phase 202.a under electrode 200.a. Similarly, the other three ECNB phases 202.b, 202.c, 202.d under electrodes 200.b, 200.c, 200.d respectively can be generated in a similar manner. For example, to generate ECNB phase 202.b under electrode 200.b, concurrent current-controlled phases 201.a, 201.c and 201.d are imposed in electrodes 200.a, 200.c and 200.d respectively and returned in electrode 200.b. The phases 201 may be anodic or cathodic which make phases 202 cathodic or anodic respectively.

As already outlined above, the current-controlled phases 201 are preferably composed of a rising phase 203, a plateau phase 204 of constant amplitude, and a falling phase 205. The rising phase 203 is preferably an exponential rising phase characterized by starting amplitudes 206, duration 207 and time constant 208, or a linear ramping phase. Either type of phase 203 minimizes the recruitment of large diameter Aβ sensory fibers associated with paresthesia. The falling phases 205 are preferably symmetrical to the rising phases 203. In an embodiment of the invention, the rising-exponential current generation can be implemented using different circuitry known in prior art, e.g. utilizing a metal-oxide semiconductor (MOS) device operating in weak inversion where the current-voltage characteristic follows an exponential relationship and amplifying such current. Also, the exponential function can be mathematically approximated using a pseudo-exponential approximation, a Taylor's approximation or a pseudo-Taylor approximation.

Furthermore, according to a preferred embodiment, each plateau phase 204 has a programmable duration 209 between 0.1 s and several seconds, preferably in the range from 0.1 s to 4.0 s. On the other hand, each rising and falling phase 203, 205 have a duration 207 with a similar programming range as plateau phase duration 209 but preferably smaller or equal to 2.0 s. Furthermore, an inter-pulse interval (IPI) 210 is provided between ECNB phases 202, which IPI 210 is preferably also programmable between 0.1 s and several seconds, and is preferably smaller or equal to 2.0 s. Finally, the amplitude of the plateau phase 204 is preferably programmable in the range of 0.1 mA to 5.0 mA. Furthermore, preferably, the ECNB phases 202 get repeated at a period equal to T which is determined by all programmable periods/durations 207, 209, and 210.

Furthermore, the Multiphase therapy according to the previously known art cannot be delivered via the traditional DC blocking capacitors used in neurostimulators for single-fault protection as passing 15 mC will require 1500 V (if we consider a typical nominal capacitor of 10 µF used in SCS).

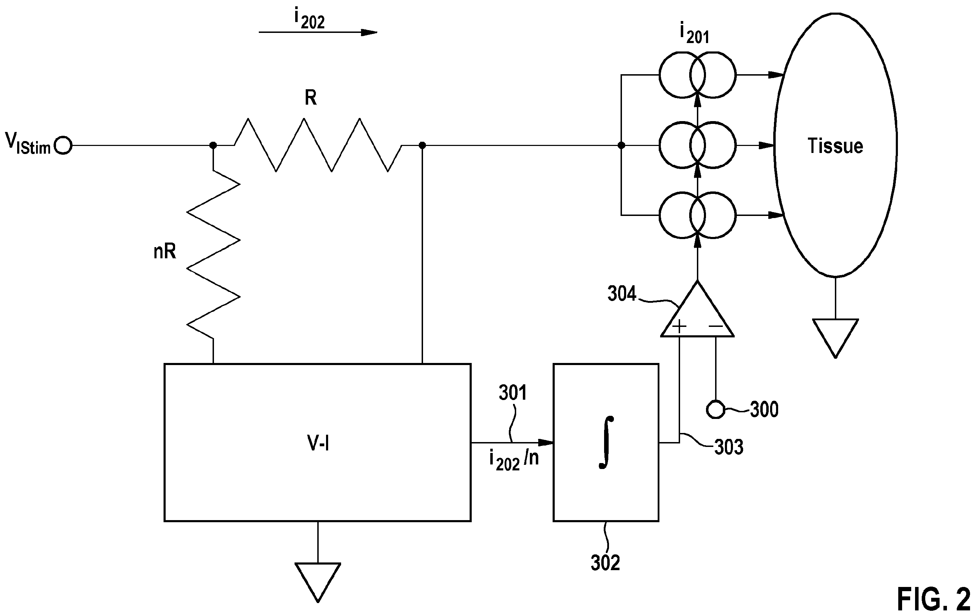

Hence in a preferred embodiment, the total charge injected during each ENCB phase 202 is measured upstream (at the programmable V_{IStim} overhead voltage required for delivering current-based stimulation) and compared to a limit value 300 to prevent electrode 200 corrosion as illustrated in Fig. 2. The ECNB phase 202 current i₂₀₂ is sensed by ratiometrically matched resistors R and n*R (R multiplied by n), where n is an integer, and the voltage generated (by this resistor sensing) converted by the voltage-to-current converter V-I to a current 301 equal to i₂₀₂/n. Such current 301 is then integrated by block 302 and its output 303 compared, via comparator 304, to limit 300 during each ECNB phase 202. If output 303 exceeds limit 300 the current sources i₂₀₁ are disconnected and a fault declared in the IPG preventing further stimulation.

Under the single-fault safe embodiment of charge metering illustrated in Fig. 2, accumulated charge caused by mismatch between the ECNB phases 202 and the corresponding phases 201 is handled by passive charge balancing between the participating electrodes 200 and tissue. Analogue switches connect each participating electrode 200 to system ground V_{SS} to perform passive charge balance. These analogue switches are driven with current limit to protect against single fault. Although Fig. 2 illustrates cathodic ECNB phases 202, the same monitoring upstream at programmable voltage V_{IStim} can be utilized when i₂₀₁ are sinking currents to implement anodic ECNB phases 202.

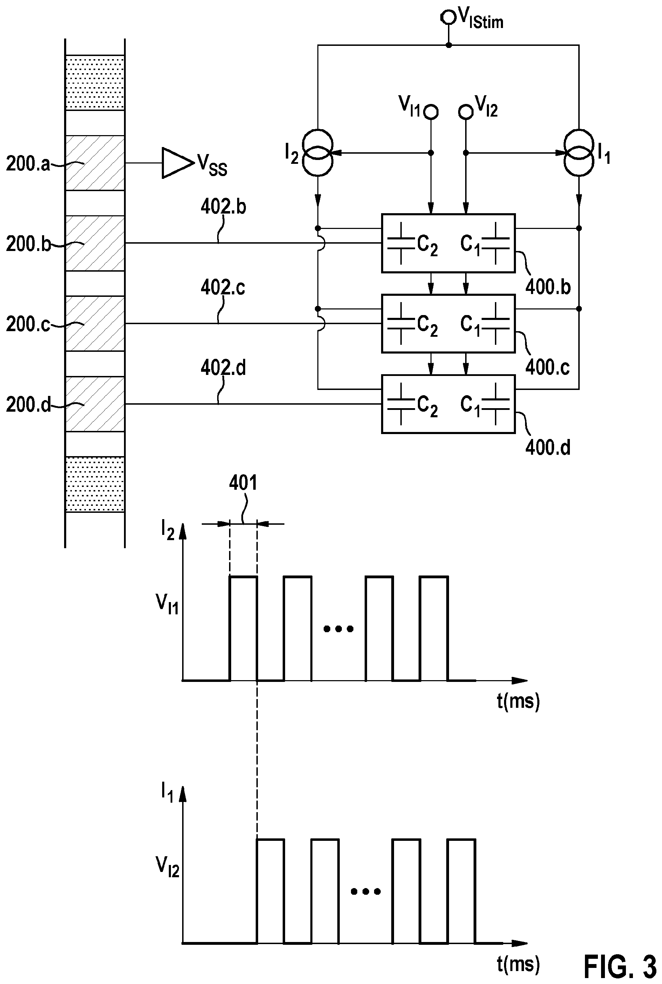

The embodiment of charge metering described above is suitable for very low frequency (sub Hz). For higher frequency (super Hz), in another preferred embodiment, the Multiphase therapy of the present invention is delivered via a switching DC blocking scheme, wherein two DC blocking capacitors C₁, C₂ per participating electrode 200 are used as illustrated in Fig. 3.

Particularly, for providing cathodic ECNB by means of electrode 200.a, the latter is preferably tied to system ground V_{SS} (e.g. negative terminal of IPG battery) and the other three electrodes 200.b, 200.c, 200.d are connected to building blocks 400.b, 400.c, 400.d, respectively. Each building block 400 includes two capacitors C₁, C₂ preferably of nominal capacitance 10 µF, and associated circuitry that permits alternating in time the connection in series with the corresponding electrode 200 via connection 402. The alternating connection of the blocking capacitors C₁, C₂ of each building block 400 is controlled by signals V_{I1} and V_{I2}. When V_{I1} is high, it turns on sourcing current I₂ and a copy of it flows through capacitor C₂ which gets connected to the corresponding electrode 200, while at the same time DC blocking capacitor C₁ is discharged through switch 505. On the other hand, when V₁₂ is high, it turns on sourcing current I₁ and a copy of it flows through capacitor C₁ which gets connected to the corresponding electrode 200, while discharging DC blocking capacitor C₂. The sum of currents circulating through electrodes 200.b, 200.c, 200.d gets returned at electrode 200.a providing the cathodic ECNB phase 202.a under description in this example.

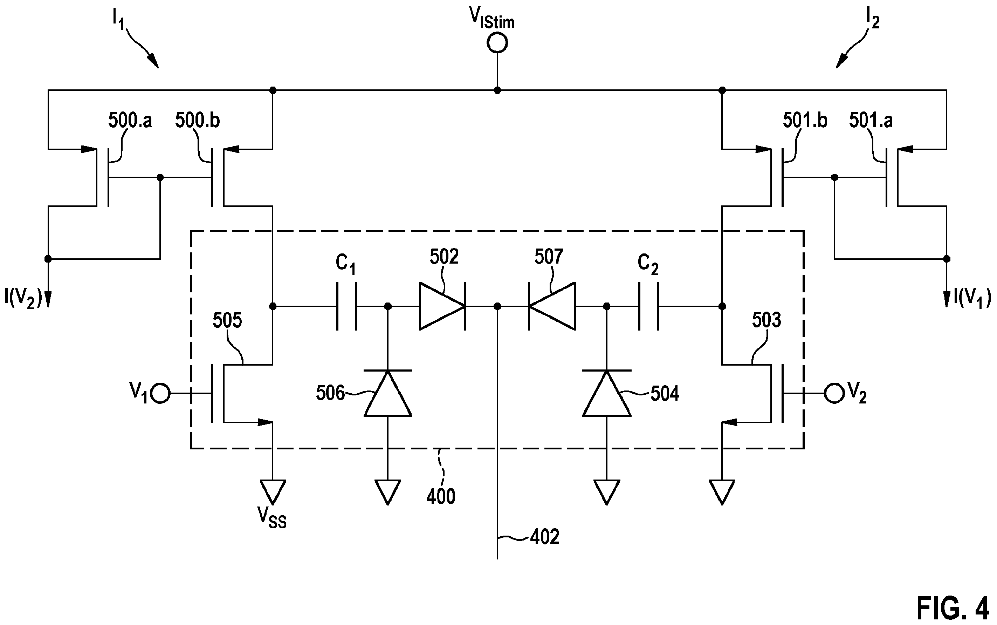

In a preferred embodiment, each capacitor C₁, C₂ is connected for a time 401 equal to preferably 500 µs (i.e. 1 kHz switching). Considering a nominal value of 10 µF, each capacitor will charge up to 0.25 V when the maximum current of 5.0 mA circulates through them. This voltage overhead will add to the other voltage overhead of the tissue-electrode capacitance itself. Particularly, the expected capacitance for the surface-modified electrode 200 can have a minimum value of about 200 µF/mm². Given a typical area of 12.5 mm² for a percutaneous SCS electrode this translates into 2.5 mF. Passing 15 mC as mentioned before will then require a 6 V overhead. Considering the ohmic drops in tissue plus the compliance voltage of the sourcing currents I₁, I₂ the programmable voltage V_{Istim} (see Fig. 4) to complete the circuit to deliver the Multiphase therapy of the present invention can have a programmable range up to about 15 V. This is in line with the programmable values required in state of the art SCS IPGs on the market. No higher voltage is required. A typical circuit to generate voltage V_{Istim} utilizes a DC-DC step up converter.

A similar block 400.a (not shown) is associated with electrode 200.a. The delivery of any other ECNB phase 202 can be described in a similar fashion as for the case of electrode 200.a. In the case of anodic ECNB phases 202, a symmetrical circuit to that of Fig. 3, i.e. sinking currents I₁, I₂ and electrode 200.a connected to voltage V_{IStim} instead in the example being described, can be employed.

A preferred implementation for block 400 is shown in Fig. 4. Other distributed DC blocking capacitor embodiments are possible and may be implemented by someone skilled in the art. Sourcing currents I₁ and I₂ are generated via voltage-controlled current references I(V₂) and I(V₁) and current mirrors 500 and 501 respectively. Diode 502 permits circulating current I₁ through DC blocking capacitor C₁ connecting to output 402 which connects to the corresponding electrode 200. When current I₁ is circulating, analogue switch 503 and diode 504 permit discharging DC blocking capacitor C₂ which was charged in a previous operation when current I₂ circulated through it. Similarly, analogue switch 505 and diode 506 permit discharging DC blocking capacitor C₁ when I₂ is circulating through DC blocking capacitor via diode 507. In an alternative embodiment, the diodes 502, 504, 506, and 507 are replaced by analogue switches controlled by signal V₁ and V₂. Any analogue switch that connects directly to tissue is preferably driven with current limit to protect against single fault.

The alternating use of capacitors C₁ and C₂ will cause a small ripple in the plateau amplitudes 204 but its effect is negligible for the purpose of nerve conduction blocking.

Different amplitudes of the plateau phase 204 may be utilized for each participating electrode 200 to create different ECNB phases 202. To handle the accumulated charge caused by mismatch, all 400 blocks of the participating electrodes (200.a, ... , 200.d in the example being described) simultaneously activate the switching of analogue switches 505, 503 to their capacitors C₁ and C₂ but without currents I₁ and I₂ circulation during at least one IPI, preferably the last IPI 211 (see Fig. 1) before the cyclic repetition of the ECNB phases 202. This implements a "switched" passive balance phase similar to those used in tonic SCS stimulation to balance charge.

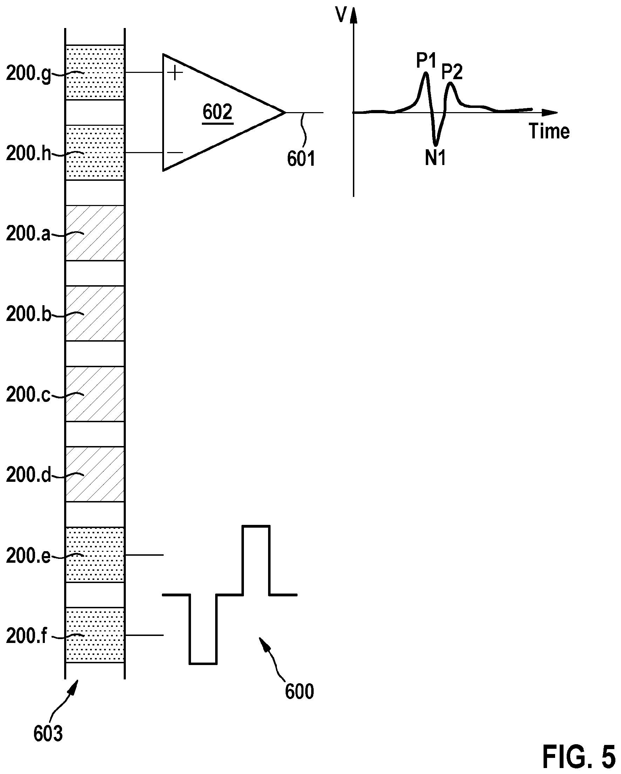

In yet another preferred embodiment of the present invention, the Multiphase therapy according to the present invention is delivered duty cycled as a nerve conduction blocking effect, and changes in neural excitability, can last for minutes once therapy is ran for a certain period and then turned off, which saves power consumption. To assess the presence of a nerve conduction blocking effect or similar changes in excitability, and with the option of adjustment of the amplitude of the plateau phase 204 in response, sub-perception biphasic pulses 600 can be injected in the two most distal(proximal) electrodes 200.e, 200.f and the presence or absence of an evoked compound action potential (ECAP) response 601 sensed by front-end 602 at the most proximal(distal) electrodes 200.g, 200.h on a same lead 603, as illustrated in Fig. 5.

In a preferred embodiment ECAP sensing occurs continuously during at least one of the IPI periods, or during the off period of the Multiphase therapy duty cycle.

The present invention allows to provide in an advantageous fashion an ultra-low-frequency (ULF) SCS. It will be apparent to those skilled in the art that numerous modifications and variations of the described examples and embodiments are possible in light of the above teaching. The disclosed examples and embodiments are presented for purposes of illustration only. Therefore, it is the intent to cover all such modifications and alternate embodiments as may come within the true scope of this invention.

**Table of Reference Signs**

| | |
|---|---|
| 1 | Neurostimulation system |
| 2 | Implantable pulse generator (IPG) |
| 3 | Percutaneous or paddle lead(s) |
| 200 | Electrode(s) |
| 200.a-200.h | Individual electrodes in a group of N electrodes |
| 201.a-201.d | Charge balancing pulses |
| 202.a-202.d | Therapeutic electric pulses (TEPs) |
| 203 | Rising phase of the pulse shape |
| 204 | Plateau phase of the pulse shape |
| 205 | Falling phase of the pulse shape |
| 206 | First current amplitude (start/end of pulse) |
| 207 | Duration of rising/falling phase |
| 208 | Time constant of exponential rising phase |
| 209 | Duration of plateau phase |
| 210 | Inter-pulse interval (IPI) |
| 211 | Last IPI before cyclic repetition |
| 300 | Limit value for total charge |
| 301 | Further current (scaled version of i202) |
| 302 | Integrator block for charge measurement |
| 303 | Output of integrator (total charge) |
| 304 | Comparator for charge limit detection |
| 400.a-400.d | Building blocks for switching DC blocking capacitors |
| 401 | Time duration for capacitor connection (e.g., 500 µs) |
| 402 | Connection to corresponding electrode |
| 500, 501 | Current mirrors |
| 502-507 | Diodes for current routing and capacitor discharge |
| 503, 505 | Analog switches for capacitor discharge |
| 600 | Sub-perception biphasic pulses |
| 601 | Evoked compound action potential (ECAP) response |
| 602 | Front-end for ECAP sensing |
| 603 | Lead for ECAP sensing |
| C1, C2 | DC blocking capacitors |
| I1, I2 | Sourcing currents |
| i₂₀₁, i₂₀₂ | Current sources |
| VI1, VI2 | Control signals for capacitor switching |
| VIStim | Programmable stimulation voltage |
| VSS | System ground (e.g., negative terminal of IPG battery) |
| R, n·R | Resistors for current sensing |

## Claims

1. A neurostimulation system (1), comprising a plurality of Z electrodes (200), wherein Z is an integer number and equal or larger than 3, and a programmable pulse generator (2) configured to deliver in a cycle via each electrode (200.a, 200.b, 200.c, 200.d) of a group of N electrodes of said plurality of Z electrodes, wherein N is an integer number being smaller or equal to Z, and wherein N is equal to or larger than 3, wherein N is equal to 3 in case Z is equal to 3, a set of electric pulses including one therapeutic electric pulse (202.a, 202.b, 202.c, 202.d), and a number of N-1 charge balancing pulses (201.a, 201.b, 201.c, 201.d), wherein the charge balancing electric pulses each have a polarity that is opposite a polarity of the therapeutic electric pulse, and
wherein the integrated average current delivered by the therapeutic electric pulse (202.a, 202.b, 202.c, 202.d) and charge balancing pulses (201.a, 201.b, 201.c, 201.d) is zero over time;
**characterized in that**
at least one of the electric pulses comprises a pulse shape comprising
- a rising phase (203) having a duration of at least 0.1s,
- a plateau phase (204) having a duration of at least 0.1 s, and
- a falling phase (205) having a duration of at least 0.1 s.

2. The neurostimulation system (1) according to claim 1, **wherein** the rising phase (203) has a duration being smaller or equal to 4 s, wherein the plateau phase (204) has a duration being smaller or equal to 4 s, and wherein the falling phase (205) has a duration being smaller or equal to 4 s.

3. The neurostimulation system (1) according to claim 2, **wherein** during the rising phase (203), a current level of the pulse shape [electric pulse] steadily rises from a first current amplitude (206) to a plateau phase (204) current amplitude, wherein the plateau phase (204) current amplitude is in the range of 0.1 mA to 5.0 mA, and wherein during the plateau phase (204), the current level of the pulse shape maintains the plateau current amplitude, and wherein during the falling phase (205), the current level of pulse shape decreases from the plateau phase (204) current amplitude to the first current amplitude (206).

4. The neurostimulation system (1) according to one of the preceding claims, wherein the integrated average current delivered by the therapeutic electric pulse (202.a, 202.b, 202.c, 202.d) and charge balancing pulses (201.a, 201.b, 201.c, 201.d) and a passive net balancing phase which compensates for charge differences between the therapeutic electric pulses and the charge balancing pulses, is zero over time.

5. The neurostimulation system (1) according to claim 2 or 3, **wherein** the programmable pulse generator (2) is configured to generate at least two successive electric pulses having said pulse shape, wherein the at least two successive electric pulses are separated by an inter-pulse-interval (210) of at least 0.1 s.

6. The neurostimulation system (1) according to claim 5, wherein the inter-pulse-interval (210) is smaller or equal than 4 s.

7. The neurostimulation system (1) according to one of the preceding claims, **wherein** the programmable pulse generator (2) is configured to generate an inter-pulse-interval (210) of between each electric pulse of said set of electric pulses, wherein the respective inter-pulse-interval (210) is in the range from 0.1 s to 4 s.

8. The neurostimulation system (1) according to one of the preceding claims, **wherein** the neurostimulation system (1) is configured to measure a total charge injected during at least a therapeutic electric pulse (202.a, 202.b, 202.c, 202.d) and to compare it to a limit value (300).

9. The neurostimulation system (1) according to claim 8, **wherein** for measuring said total charge the neurostimulation system (1) is configured to sense an electric current (i₂₀₂) of at least a therapeutic electric pulse (202.a, 202.b, 202.c, 202.d) using a resistor having a resistance R and a further resistor having a resistance n*R, wherein n is an integer number, and a voltage converted by a voltage-to-current converter (V-I) to a further current (301) being equal to said electric current (i₂₀₂) divided by said integer number n, wherein the neurostimulation system (1) is further configured to integrate said further current (301) to generate an output (303) corresponding to said total charge, and to compare the output (303) to said limit value (300).

10. The neurostimulation system (1) according to claim 8 or 9, wherein the neurostimulation system (1) is configured to disconnect current sources (i₂₀₁) providing the charge balancing electric pulses (201.a, 201.b, 201.c, 201.d) and to declare a fault to prevent further stimulation.

11. The neurostimulation system (1) according to one of the claims 1 to 7, wherein for providing a therapeutic electric pulse (202.a) by means of an electrode (200.a) of said group of N electrodes, said electrode (200.a) is tied to system ground (Vss) and the remaining electrodes (200.b, 200.c, 200.d) of said group of N electrodes are each connected to an associated building block (400.b, 400.c, 400.d), each building block comprising a first and a second blocking capacitor (C₁, C₂) and circuitry that permits alternating in time a connection in series with the corresponding remaining electrode (200.b, 200.c, 200.d).

12. The neurostimulation system (1) according to claim 11, wherein the neurostimulation system (1) is configured to control the alternating connection of the blocking capacitors C₁, C₂ of each building block by a first and a second signal (V_{I1}, V_{I2}) assuming a high value and a low value, respectively, wherein when the first signal (Vn) assumes its high value, it turns on a sourcing current (I₂) and a copy of it flows through second blocking capacitor (C₂) which gets connected to the corresponding remaining electrode (200.b, 200.c, 200.d), while discharging the first blocking capacitor (C₁), and wherein when the second signal (V_{I2}) assumes its high value, it turns on a further sourcing current (I₁) and a copy of it flows through first blocking capacitor (C₁) which gets connected to the corresponding remaining electrode (200.b, 200.c, 200.d), while discharging the second blocking capacitor (C₂), wherein a sum of currents circulating through the remaining electrodes (200.b, 200.c, 200.d) gets returned at said electrode (200.a) providing the therapeutic electric pulse (202.a)

13. The neurostimulation system (1) according to one of the preceding claims, **wherein** said pulse shape starts with the rising phase (203), followed by a plateau phase (204), followed by a falling phase (205) for an anodic pulse. Alternatively, said pulse shape starts with the falling phase, followed by a plateau phase, followed by a rising phase for a cathodic pulse.

14. The neurostimulation system (1) according to one of the preceding claims, **wherein** each electrode (200.a, 200.b, 200.c, 200.d) undergoes the therapeutic electric pulse (202.a, 202.b, 202.c, 202.d) in a cyclic fashion with charge balancing pulses (201.a, 201.b, 201.c, 201.d) of opposite polarity, and wherein the current of each therapeutic electric pulse is substantially returned by the charge balancing pulses in the other N-1 electrodes.

15. The neurostimulation system (1) according to one of the preceding claims, **wherein** the neurostimulation system (1) is a spinal cord stimulation system, a deep brain stimulation system or a peripheral nerve stimulation system.

## Patentansprüche

1. Neurostimulationssystem (1), umfassend eine Vielzahl von Z Elektroden (200), wobei Z eine ganze Zahl ist und gleich oder größer als 3 ist, und einen programmierbaren Impulsgenerator (2), der dazu konfiguriert ist, in einem Zyklus über jede Elektrode (200.a, 200.b, 200.c, 200.d) einer Gruppe von N Elektroden der Vielzahl von Z Elektroden, wobei N eine ganze Zahl kleiner oder gleich Z ist und wobei N gleich oder größer als 3 ist, wobei N gleich 3 ist, wenn Z gleich 3 ist, einen Satz von elektrischen Impulsen, einschließlich eines therapeutischen elektrischen Impulses (202.a, 202.b, 202.c, 202.d) und einer Anzahl von N-1 Ladungsausgleichsimpulsen (201.a, 201.b, 201.c, 201.d), zu liefern, wobei die elektrischen Ladungsausgleichsimpulse jeweils eine Polarität aufweisen, die einer Polarität des therapeutischen elektrischen Impulses entgegengesetzt ist, und
wobei der integrierte durchschnittliche Strom, der von dem therapeutischen elektrischen Impuls (202.a, 202.b, 202.c, 202.d) und den Ladungsausgleichsimpulsen (201.a, 201.b, 201.c, 201.d) geliefert wird, im Zeitverlauf null ist;
**dadurch gekennzeichnet, dass**
mindestens einer von den elektrischen Impulsen eine Impulsform umfasst, die Folgendes umfasst:
- eine Anstiegsphase (203) mit einer Dauer von mindestens 0,1 s,
- eine Plateauphase (204) mit einer Dauer von mindestens 0,1 s und
- eine Abstiegsphase (205) mit einer Dauer von mindestens 0,1 s.

2. Neurostimulationssystem (1) nach Anspruch 1, **wobei** die Anstiegsphase (203) eine Dauer aufweist, die kleiner oder gleich 4 s ist, wobei die Plateauphase (204) eine Dauer aufweist, die kleiner oder gleich 4 s ist, und wobei die Abstiegsphase (205) eine Dauer aufweist, die kleiner oder gleich 4 s ist.

3. Neurostimulationssystem (1) nach Anspruch 2, **wobei** während der Anstiegsphase (203) ein Stromniveau der Impulsform [elektrischer Impuls] stetig von einer ersten Stromamplitude (206) zu einer Stromamplitude der Plateauphase (204) ansteigt, wobei die Stromamplitude der Plateauphase (204) im Bereich von 0,1 mA bis 5,0 mA liegt und wobei während der Plateauphase (204) das Stromniveau der Impulsform die Stromamplitude der Plateauphase beibehält, und wobei während der Abstiegsphase (205) das Stromniveau der Impulsform von der Stromamplitude der Plateauphase (204) zu der ersten Stromamplitude (206) abnimmt.

4. Neurostimulationssystem (1) nach einem der vorhergehenden Ansprüche, wobei der integrierte durchschnittliche Strom, der von dem therapeutischen elektrischen Impuls (202.a, 202.b, 202.c, 202.d) und den Ladungsausgleichsimpulsen (201.a, 201.b, 201.c, 201.d) geliefert wird, und eine passive Nettoausgleichsphase, die Ladungsdifferenzen zwischen den therapeutischen elektrischen Impulsen und den Ladungsausgleichsimpulsen ausgleicht, im Zeitverlauf null ist.

5. Neurostimulationssystem (1) nach Anspruch 2 oder 3, **wobei** der programmierbare Impulsgenerator (2) dazu konfiguriert ist, mindestens zwei aufeinanderfolgende elektrische Impulse mit der Impulsform zu generieren, wobei die mindestens zwei aufeinanderfolgenden elektrischen Impulse durch ein Zwischenimpulsintervall (210) von mindestens 0,1 s getrennt sind.

6. Neurostimulationssystem (1) nach Anspruch 5, wobei das Zwischenimpulsintervall (210) kleiner oder gleich 4 s ist.

7. Neurostimulationssystem (1) nach einem der vorhergehenden Ansprüche, **wobei** der programmierbare Impulsgenerator (2) dazu konfiguriert ist, ein Zwischenimpulsintervall (210) zwischen jedem elektrischen Impuls des Satzes von elektrischen Impulsen zu generieren, wobei das jeweilige Zwischenimpulsintervall (210) im Bereich von 0,1 s bis 4 s liegt.

8. Neurostimulationssystem (1) nach einem der vorhergehenden Ansprüche, **wobei** das Neurostimulationssystem (1) dazu konfiguriert ist, eine Gesamtladung zu messen, die während mindestens eines therapeutischen elektrischen Impulses (202.a, 202.b, 202.c, 202.d) injiziert wurde, und sie mit einem Grenzwert (300) zu vergleichen.

9. Neurostimulationssystem (1) nach Anspruch 8, **wobei** zum Messen der Gesamtladung das Neurostimulationssystem (1) dazu konfiguriert ist, einen elektrischen Strom (i₂₀₂) von mindestens einem therapeutischen elektrischen Impuls (202.a, 202.b, 202.c, 202.d) unter Verwendung eines Widerstands mit einem Widerstandswert R und eines weiteren Widerstands mit einem Widerstandswert n*R, wobei n eine ganze Zahl ist, und eine Spannung, die durch einen Spannung-zu-Strom-Wandler (V-I) in einen weiteren Strom (301) umgewandelt wird, der gleich dem elektrischen Strom (i₂₀₂), geteilt durch die ganze Zahl n, ist, zu erfassen, wobei das Neurostimulationssystem (1) ferner dazu konfiguriert ist, den weiteren Strom (301) zu integrieren, um eine Ausgabe (303) zu generieren, die der Gesamtladung entspricht, und die Ausgabe (303) mit dem Grenzwert (300) zu vergleichen.

10. Neurostimulationssystem (1) nach Anspruch 8 oder 9, wobei das Neurostimulationssystem (1) dazu konfiguriert ist, Stromquellen (i₂₀₁) zu trennen, die die elektrischen Ladungsausgleichsimpulse (201.a, 201.b, 201.c, 201.d) bereitstellen, und einen Fehler anzugeben, um eine weitere Stimulation zu verhindern.

11. Neurostimulationssystem (1) nach einem der Ansprüche 1 bis 7, wobei zum Bereitstellen eines therapeutischen elektrischen Impulses (202.a) mittels einer Elektrode (200.a) der Gruppe von N Elektroden die Elektrode (200.a) mit Systemmasse (Vss) verbunden ist und die übrigen Elektroden (200.b, 200.c, 200.d) der Gruppe von N Elektroden jeweils mit einem zugehörigen Baustein (400.b, 400.c, 400.d) verbunden sind, wobei jeder Baustein einen ersten und einen zweiten Sperrkondensator (C₁, C₂) und Schaltung umfasst, die ermöglicht, dass im Zeitverlauf eine Reihenverbindung mit der entsprechenden verbleibenden Elektrode (200.b, 200.c, 200.d) gewechselt wird.

12. Neurostimulationssystem (1) nach Anspruch 11, wobei das Neurostimulationssystem (1) dazu konfiguriert ist, die wechselnde Verbindung der Sperrkondensatoren C₁, C₂ jedes Bausteins durch ein erstes und ein zweites Signal (V₁₁, V₁₂) zu sperren, wobei ein hoher Wert bzw. ein niedriger Wert angenommen werden, wobei, wenn das erste Signal (V₁₁) seinen hohen Wert annimmt, es einen Quellstrom (I₂) einschaltet und eine Kopie davon durch den zweiten Sperrkondensator (C₂) strömt, der mit der entsprechenden verbleibenden Elektrode (200.b, 200.c, 200.d) verbunden wird, während der erste Sperrkondensator (C₁) entladen wird, und wobei, wenn das zweite Signal (V_{I2}) seinen hohen Wert annimmt, es einen weiteren Quellstrom (I₁) einschaltet und eine Kopie davon durch den ersten Sperrkondensator (C₁) strömt, der mit der entsprechenden verbleibenden Elektrode (200.b, 200.c, 200.d) verbunden wird, während der zweite Sperrkondensator (C₂) entladen wird, wobei eine Summe aus Strömen, die durch die verbleibenden Elektroden (200.b, 200.c, 200.d) zirkulieren, an der Elektrode (200.a) zurückgegeben wird, wobei der therapeutische elektrische Impuls (202.a) bereitgestellt wird.

13. Neurostimulationssystem (1) nach einem der vorhergehenden Ansprüche, **wobei** die Impulsform mit der Anstiegsphase (203) beginnt, gefolgt von einer Plateauphase (204), gefolgt von einer Abstiegsphase (205) für einen anodischen Impuls. Alternativ beginnt die Impulsform mit der Abstiegsphase, gefolgt von einer Plateauphase, gefolgt von einer Anstiegsphase für einen kathodischen Impuls.

14. Neurostimulationssystem (1) nach einem der vorhergehenden Ansprüche, wobei jede Elektrode (200.a, 200.b, 200.c, 200.d) dem therapeutischen elektrischen Impuls (202.a, 202.b, 202.c, 202.d) auf eine zyklische Weise mit Ladungsausgleichsimpulsen (201.a, 201.b, 201.c, 201.d) mit entgegengesetzter Polarität unterzogen wird und wobei der Strom jedes therapeutischen elektrischen Impulses im Wesentlichen durch die Ladungsausgleichimpulse in den anderen N-1 Elektroden zurückgegeben wird.

15. Neurostimulationssystem (1) nach einem der vorhergehenden Ansprüche, **wobei** das Neurostimulationssystem (1) ein Rückmark-Stimulationssystem, ein System zur tiefen Hirnstimulation oder ein Stimulationssystem des peripheren Nervensystems ist.

## Revendications

1. Système de neurostimulation (1), comprenant une pluralité de Z électrodes (200), dans lequel Z représente un nombre entier supérieur ou égal à 3, et un générateur d'impulsions programmable (2) configuré pour distribuer en un cycle par l'intermédiaire de chaque électrode (200.a, 200.b, 200.c, 200.d) d'un groupe de N électrodes de ladite pluralité de Z électrodes, dans lequel N représente un nombre entier inférieur ou égal à Z, et dans lequel N est supérieur ou égal à 3, dans lequel N est égal à 3 dans le cas où Z est égal à 3, un ensemble d'impulsions électriques incluant une impulsion électrique thérapeutique (202.a, 202.b, 202.c, 202.d), et un nombre de N-1 impulsions d'équilibrage de charge (201.a, 201.b, 201.c, 201.d), dans lequel les impulsions électriques d'équilibrage de charge ont chacune une polarité qui est opposée à une polarité de l'impulsion électrique thérapeutique, et
dans lequel le courant moyen intégré distribué par l'impulsion électrique thérapeutique (202.a, 202.b, 202.c, 202.d) et les impulsions d'équilibrage de charge (201.a, 201.b, 201.c, 201.d) est zéro en fonction du temps ;
**caractérisé en ce que**
au moins l'une des impulsions électriques comprend une forme d'impulsion comprenant
- une phase ascendante (203) ayant une durée d'au moins 0,1 s,
- une phase de plateau (204) ayant une durée d'au moins 0,1 s, et
- une phase descendante (205) ayant une durée d'au moins 0,1 s.

2. Système de neurostimulation (1) selon la revendication 1, **dans lequel** la phase ascendante (203) a une durée inférieure ou égale à 4 s, dans lequel la phase de plateau (204) a une durée inférieure ou égale à 4 s, et dans lequel la phase descendante (205) a une durée inférieure ou égale à 4 s.

3. Système de neurostimulation (1) selon la revendication 2, **dans lequel** pendant la phase ascendante (203), un niveau de courant de la forme d'impulsion [impulsion électrique] croît régulièrement depuis une première amplitude de courant (206) jusqu'à une amplitude de courant de phase de plateau (204), dans lequel l'amplitude de courant de phase de plateau (204) est dans la plage de 0,1 mA à 5,0 mA, et dans lequel pendant la phase de plateau (204), le niveau de courant de la forme d'impulsion conserve l'amplitude de courant de plateau, et dans lequel pendant la phase descendante (205), le niveau de courant de forme d'impulsion décroît depuis l'amplitude de courant de phase de plateau (204) jusqu'à la première amplitude de courant (206).

4. Système de neurostimulation (1) selon l'une des revendications précédentes, dans lequel le courant moyen intégré distribué par l'impulsion électrique thérapeutique (202.a, 202.b, 202.c, 202.d) et les impulsions d'équilibrage de charge (201.a, 201.b, 201.c, 201.d) et une phase d'équilibrage net passif qui compense des différences de charge entre les impulsions électriques thérapeutiques et les impulsions d'équilibrage de charge, est de zéro sur le temps.

5. Système de neurostimulation (1) selon la revendication 2 ou 3, **dans lequel** le générateur d'impulsions programmable (2) est configuré pour générer au moins deux impulsions électriques successives ayant ladite forme d'impulsion, dans lequel les au moins deux impulsions électriques successives sont séparées par un intervalle inter-impulsions (210) d'au moins 0,1 s.

6. Système de neurostimulation (1) selon la revendication 5, dans lequel l'intervalle inter-impulsions (210) est inférieur ou égal à 4 s.

7. Système de neurostimulation (1) selon l'une des revendications précédentes, **dans lequel** le générateur d'impulsions programmable (2) est configuré pour générer un intervalle inter-impulsions (210) entre chaque impulsion électrique dudit ensemble d'impulsions électriques, dans lequel l'intervalle inter-impulsions (210) respectif est dans la plage de 0,1 s à 4 s.

8. Système de neurostimulation (1) selon l'une des revendications précédentes, **dans lequel** le système de neurostimulation (1) est configuré pour mesurer une charge totale injectée pendant au moins une impulsion électrique thérapeutique (202.a, 202.b, 202.c, 202.d) et pour la comparer à une valeur limite (300).

9. Système de neurostimulation (1) selon la revendication 8, dans **lequel** pour mesurer ladite charge totale le système de neurostimulation (1) est configuré pour détecter un courant électrique (i₂₀₂) d'au moins une impulsion électrique thérapeutique (202.a, 202.b, 202.c, 202.d) en utilisant une résistance de valeur de résistance R et une résistance supplémentaire ayant une valeur de résistance n*R, dans lequel n représente un nombre entier, et une tension convertie par un convertisseur tension-courant (V-I) en un courant supplémentaire (301) égal audit courant électrique (i₂₀₂) divisé par ledit nombre entier n, dans lequel le système de neurostimulation (1) est en outre configuré pour intégrer ledit courant supplémentaire (301) afin de générer une sortie (303) correspondant à ladite charge totale, et pour comparer la sortie (303) à ladite valeur limite (300).

10. Système de neurostimulation (1) selon la revendication 8 ou 9, dans lequel le système de neurostimulation (1) est configuré pour déconnecter les sources de courant (i₂₀₁) fournissant les impulsions électriques d'équilibrage de charge (201.a, 201.b, 201.c, 201.d) et pour déclarer un défaut afin d'empêcher toute stimulation supplémentaire.

11. Système de neurostimulation (1) selon l'une des revendications 1 à 7, dans lequel pour fournir une impulsion électrique thérapeutique (202.a) au moyen d'une électrode (200.a) dudit groupe de N électrodes, ladite électrode (200.a) est rattachée à la terre du système (Vₛₛ) et les électrodes restantes (200.b, 200.c, 200.d) dudit groupe de N électrodes sont chacune connectée à un bloc de construction (400.b, 400.c, 400.d) associé, chaque bloc de construction comprenant un premier et un second condensateur de blocage (C₁, C₂) et un circuit qui permet d'alterner dans le temps une connexion en série avec l'électrode restante (200.b, 200.c, 200.d) correspondante.

12. Système de neurostimulation (1) selon la revendication 11, dans lequel le système de neurostimulation (1) est configuré pour commander la connexion alternée des condensateurs de blocage C₁, C₂ de chaque bloc de construction par un premier et un second signal (V_{I1}, V_{I2}) qui prennent respectivement une valeur haute et une valeur basse, dans lequel lorsque le premier signal (V_{I1}) prend sa valeur haute, il allume un courant de source (I₂) et une copie de celui-ci traverse un second condensateur de blocage(C₂) qui est connecté à l'électrode restante (200.b, 200.c, 200.d) correspondante, tout en déchargeant le premier condensateur de blocage (C₁), et dans lequel lorsque le second signal (V_{I2}) prend sa valeur haute, il allume un courant de source (I₁) supplémentaire et une copie de celui-ci traverse un premier condensateur de blocage (C₁) qui est connecté à l'électrode restante (200.b, 200.c, 200.d) correspondante, tout en déchargeant le second condensateur de blocage (C₂), dans lequel une somme de courants circulant à travers les électrodes restantes (200.b, 200.c, 200.d) est renvoyé à ladite électrode (200.a) fournissant l'impulsion électrique thérapeutique (202.a)

13. Système de neurostimulation (1) selon l'une des revendications précédentes, **dans lequel** ladite forme d'impulsion commence par la phase ascendante (203), suivie par une phase de plateau (204), suivie par une phase descendante (205) pour une impulsion anodique. De manière alternative, ladite forme d'impulsion commence par la phase descendante, suivie par une phase de plateau, suivi par une phase ascendante pour une impulsion cathodique.

14. Système de neurostimulation (1) selon l'une des revendications précédentes, **dans lequel** chaque électrode (200.a, 200.b, 200.c, 200.d) subit l'impulsion électrique thérapeutique (202.a, 202.b, 202.c, 202.d) d'une façon cyclique avec des impulsions d'équilibrage de charge (201.a, 201.b, 201.c, 201.d) de polarité opposée, et dans lequel le courant de chaque impulsion électrique thérapeutique est essentiellement renvoyé par les impulsions d'équilibrage de charge dans les autres N-1 électrodes.

15. Système de neurostimulation (1) selon l'une des revendications précédentes, **dans lequel** le système de neurostimulation (1) est un système de stimulation de moelle épinière, un système de stimulation cérébrale profonde ou un système de stimulation de nerfs périphériques.
